# EUROPEAN PATENT SPECIFICATION

(11) **EP 0 649 851 B1**
(45) Date of publication and mention of the grant of the patent: **12.05.1999**
(21) Application number: 94116502.9
(22) Date of filing: 19.10.1994
(51) Int. Cl.: C07F 15/00, B01J 31/20, B01J 31/40, C07C 45/50

(54) **Process for preparation of hydridocarbonyltris (triorganophosphorus) rhodium compound**
Verfahren zur Herstellung einer Hydridocarbonyltris(triorganophosphor)-Rhodium-Verbindung
Procédé de préparation d'un composé hydridocarbonyl tris(triorganophosphore)rhodium

(30) Priority: 20.10.1993 KR 9322084
(43) Date of publication of application: 26.04.1995
(73) Proprietor: LUCKY LTD., Seoul 150-721 (KR)
(72) Inventor: Lee, Sang Moo, Youseong-ku, Daejeon (KR); Choi, Jung Uk, Youseong-ku, Daejeon (KR); Jung, Seong Moon, Joong-ku, Daejeon (KR); Kim, Jin Do, Youseong-ku, Daejeon (KR); Lee, Kee Houk, Youseong-ku, Daejeon (KR)
(74) Representative: Kador & Partner

(56) References cited:
- EP-A- 0 055 487
- EP-A- 0 083 094
- EP-A- 0 348 833
- GB-A- 2 085 747

## Description

### FIELD OF THE INVENTION

This invention relates to a process for the direct preparation of hydridocarbonyl tris(triorganophosphorus)rhodium compounds from the rhodium complex catalyst-containing distillation residue which is derived from the hydroformylation reaction mixture containing a deactivated rhodium complex catalyst. More specifically, the present invention pertains to a process for direct preparation of hydridocarbonyl tris(triorganophosphorus)rhodium compounds in high yield starting from the rhodium complex catalyst-containing distillation residue of the hydroformylation reaction mixture, which is treated with an oxidizing agent, which comprises the following three synthetic steps: i) synthesis of a rhodium carbonyl compound; ii) preparation of a hydridocarbonyl tris(triorganophosphorus)rhodium complex; and iii) post-treatment with carbon monoxide and hydrogen.

### BACKGROUND OF INVENTION

Hydridocarbonyl tris(triorganophosphorus)rhodium complex has been widely used as a catalyst in numerous processes, for example, hydrogen atom exchange reaction, isomerization, carbonylation and hydroformylation of olefins. Particularly, it has excellent catalytic activity and selectivity in hydroformylation of olefins and thus has been widely used in commercial processes.

In the prior art, hydridocarbonyl tris(triorganophosphorus) rhodium compounds have been prepared according to several methods starting from, for example, rhodium trichlorohydrate(RhCl₃ · 3H₂O), halocarbonyl bis(triorganophosphorus)rhodium or spent rhodium catalyst contained in the distillation residue of the hydroformylation reaction mixture.

According to the method using halocarbonyl bis(triorganophosphorus)rhodium as a starting material, hydridocarbonyl tris(triorganophosphorus)rhodium can be prepared by reducing the starting material with hydrazine in ethanol solvent as disclosed in J. Amer. Chem. Soc., 85, 3500, 1963 or by reacting chlorocarbonyl bis(triphenylphosphine)rhodium with sodium tetrahydroborate (NaBH₄) or with a triethyl amine and hydrogen under an ethanol solvent which contains an excessive triphenylphosphine, as disclosed in Chem. Commun., 305, 1967.

The method starting from rhodium trichlorohydrate can be conducted by reacting rhodium trichlorohydrate with KOH and HCHO under an ethanol solvent containing triphenylphosphine to obtain hydridocarbonyl tris(triorganophosphorus)rhodium in the yield of 94%, as disclosed in Inorg. Synth., 28, 81, 1990.

In addition, hydridocarbonyl tris(triorganophosphorus)rhodium compounds can be prepared by using the spent rhodium complex catalyst derived from the distillation residue of hydroformylation reaction mixture. However, this method is much more difficult than the above methods which use a reagent of pure chemical grade. Furthermore, since such rhodium complex catalyst is deactivated through various routes during hydroformylation reaction, it is very difficult to convert into hydridocarbonyl tris(triorganophosphorus)rhodium according to conventional methods.

EP 0,055,487 discloses a method for preparation of hydridocarbonyl tris(triorganophosphorus)rhodium in the yield of 88.6% by treating the distillation residue of the hydroformylation reaction mixture with HCl, DMF, etc., to prepare halocarbonyl bis(triorganophosphorus)rhodium which is then hydrogenated with sodium tetrahydroborate(NaBH₄).

According to GB-A-2,085,747, recovery of a Gp 8 noble metal solid complex comprises bringing an organic compound-containing solution containing therein a Group 8 noble metal complex having a tertiary organophosphorous compound as a ligand into contact with an oxidizing agent in the presence of a free tertiary organophosphorous compound, an organic polar solvent, water, and a basic substance to precipitate a solid complex of the Group 8 noble metal.

According to EP-A-348,833, a similar technique for recovery of rhodium involves the use of carbon monoxide to form a water-soluble complex with rhodium.

US Patent Specification No. 4,446,074 discloses the synthesis of hydridocarbonyl tris(triorganophosphorus)rhodium by treating the distillation residue of the hydroformylation reaction mixture with KOH in ethanol solvent and then subsequently treating the product with synthesis gas. However, the recovery yield of rhodium according to this method is as low as 48%.

In addition, US Patent Specification No. 4,113,754 discloses a process for preparation of hydridocarbonyl tris(triorganophosphine)rhodium which comprises treating the distillation residue of the hydroformylation reaction mixture with a mineral acid containing oxygen and a peroxide compound to obtain an aqueous solution of water-soluble rhodium salt, subjecting the obtained aqueous solution of water-soluble rhodium salt to a cation exchange resin to recover the rhodium compound, treating the recovered rhodium compound with an aqueous hydrochloric acid solution to obtain a hexachloridate solution, and reacting the hexachloridate solution with a tertiary phosphine and carbon monoxide in the presence of a water-soluble organic solvent to obtain chlorocarbonyl bis(triorganophosphine)rhodium which is then hydrogenated. However, this process requires the second aqueous phase and needs excessive mineral acid and hydrogen chloride aqueous solution.

Thus, the present inventors have extensively studied to develop a method for preparing hydridocarbonyl tris(triorganophosphorus)rhodium compounds in high yield from rhodium compounds, which are contained in the distillation residue of hydroformylation reaction mixture, without any disadvantages involved in the prior methods as described above. As a result, we have identified that hydridocarbonyl tris(triorganophosphorus)rhodium compounds can be prepared in high yield directly from the rhodium catalyst-containing distillation residue of the hydroformylation reaction mixture in the manner of an organic one-phase reaction without isolation of any intermediate product, according to the process of the present invention, as defined below.

Therefore, it is an object of the present invention to provide an organic one-phase process for preparing hydridocarbonyl tris(triorganophosphorus)rhodium compounds directly from the rhodium-containing distillation residue of the hydroformylation reaction mixture.

It is another object of the present invention to provide a process for preparing hydridocarbonyl tris(triorganophosphorus) rhodium compounds from the rhodium-containing distillation residue of the hydroformylation reaction mixture, wherein the rhodium component in the distillation residue is derived from a rhodium complex catalyst deactivated during hydroformylation, characterized in that the deactivated rhodium complex is converted into a rhodium carbonyl compound and the obtained rhodium carbonyl compound is reacted, without isolation, directly with an alkali metal hydroxide, formaldehyde and a triorganophosphorus ligand in the presence of a hydroxyl group-containing compound to produce a hydridocarbonyl tris(triorganophosphorus)rhodium compound which is subsequently reacted with carbon monoxide and then with hydrogen to prepare the desired hydridocarbonyl tris(triorganophosphorus)rhodium compound in high yield.

Further, it is still another object of the present invention to provide a process for the direct preparation of hydridocarbonyl tris(triorganophosphorus)rhodium compounds according to an organic one-phase reaction without using any halogen compound and isolating any intermediate product in each step.

The foregoing has outlined some of the more pertinent objects of the present invention. These objects should be construed to be merely illustrative of some of the more pertinent features and applications of the invention. Other many beneficial results can be obtained by applying the disclosed invention in a different manner or modifying the invention within the scope of the disclosure. Accordingly, other objects and a more thorough understanding of the invention may be had by referring to the disclosure of invention, in addition to the scope of the invention defined by the claims.

### DETAILED DESCRIPTION OF THE INVENTION

In one aspect, the present invention relates to a process for preparation of hydridocarbonyl tris(triorganophosphorus)rhodium directly from the rhodium-containing distillation residue of the hydroformylation reaction mixture in the manner of an organic one-phase reaction.

The hydroformylation reaction is a well known process, which is a catalytic method for the conversion of an olefin with carbon monoxide and hydrogen into an aldehyde product having one more carbon atoms than the starting olefin by means of a rhodium complex catalyst. The rhodium complex catalyst is gradually deactivated as the hydroformylation reaction progresses. In this reaction, the extent of deactivation of the catalyst depends on the reaction time and condition. Ultimately, the deactivated rhodium complex catalyst, which is determined as having no further substantial commercial value, is concentrated together with the hydroformylation reaction medium by vacuum distillation. The distillation residue of this process is generally referred as a spent hydroformylation reaction medium. Accordingly, it should be understood that the term "the spent hydroformylation reaction medium" used hereinafter has the same meaning as the distillation residue of the hydroformylation reaction mixture used in the present invention as the starting material. Since the rhodium compound is a very expensive noble metal catalyst, the spent hydroformylation reaction medium containing the deactivated rhodium catalyst should be treated according to a suitable manner to recover the rhodium compound for its reuse.

The spent hydroformylation reaction medium employed as the starting material in the present invention comprises a substantially deactivated rhodium complex catalyst, an aldehyde product, a high boiling aldehyde condensation by-product and a free triorganophosphorus compound. It is more preferable that the content of a low boiling aldehyde in the spent hydroformylation reaction medium is as low as possible because the low boiling aldehyde can dissolve hydridocarbonyl tris (triorganophosphorus) rhodium, and therefore, causes a decrease in the recovery of rhodium compounds.

The concentration of rhodium in the spent hydroformylation reaction medium which is employed as the starting material in the present invention is generally about 1,000-50,000 ppm, preferably about 1,500-30,000 ppm. The rhodium concentration lower than 1,500ppm has no commercial merit, since the use of a larger reaction vessel or the repeated operation using a common reaction vessel will be required in order to convert the spent hydroformylation reaction medium into hydridocarbonyl tris(triorganophosphorus) rhodium in the industrial scale. When the rhodium concentration is higher than 30,000ppm, the rhodium catalyst will form a great cluster during the spent hydroformylation reaction medium is concentrated, and thus is difficult to recover.

Although the content of triorganophosphorus compound in the spent hydroformylation reaction medium used as the starting material in the present invention is preferably as low as possible, it is generally 1-40 wt%, more preferably 1-30 wt%.

The spent hydroformylation reaction medium which is used as the starting material in the present invention should be contacted with an oxidizing agent such as oxygen or air at the temperature of from 60°C to 120°C and at the pressure of 0.101325 to 1.01325 MPa (1 atmosphere to 10 atmospheres) for a sufficient period before it is used as the starting material in the process according to the present invention. According to this procedure, the substantially deactivated rhodium complex is converted into an easily revivable form. Although it has been known that the rhodium catalyst is gradually deactivated over a long period when the catalyst is used in the hydroformylation reaction, a definite structure of the deactivated rhodium complex is still not established. However, it is supposed that the deactivated rhodium catalyst is partially reactivated or converted into the easily revivable form, according to the treatment with an oxidizing agent such as oxygen or air.

For example, when the hydroformylation reaction is carried out using a triphenylphosphine ligand in the presence of a rhodium complex catalyst, a diphenylphosphido-bridged rhodium cluster compound is formed to deactivate the rhodium catalyst. Then, as can be seen from the following reaction scheme [I], the diphenylphosphido-bridged rhodium cluster compound is treated with the oxidizing agent such as oxygen to oxidize the phosphine bridge and such oxidation weakens the binding force between rhodium and phosphine and therefore makes the reactivation and/or revival of the rhodium catalyst easy.

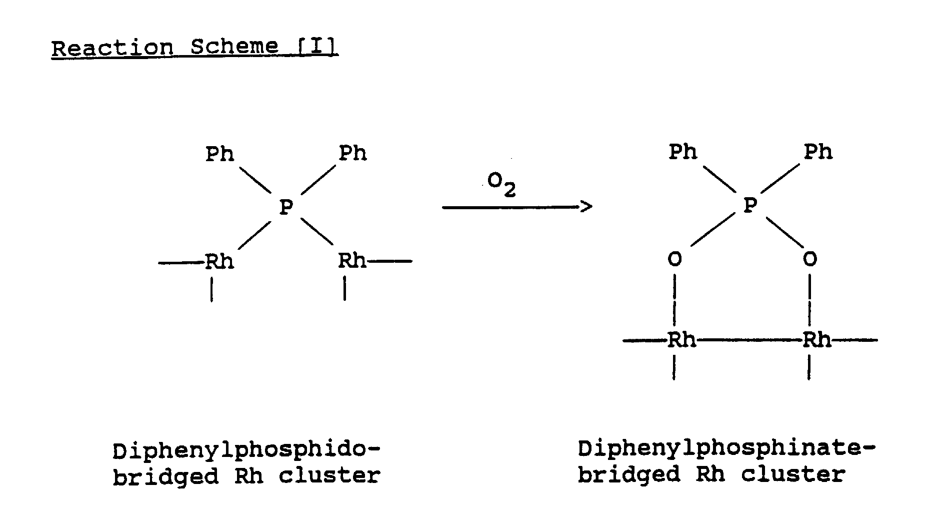

More specifically, the present invention relates to a process for preparation of hydridocarbonyl tris(triorganophosphorus) rhodium compounds in the high yield starting from the spent hydroformylation reaction medium, which contains the deactivated rhodium complex catalyst, according to the organic one-phase reaction without isolation of the intermediate product in each step, which comprises the following three steps: i) synthesis of a rhodium carbonyl complex, ii) synthesis of a hydridocarbonyl tris(triorganophosphorus)rhodium, and iii) a post-treatment with carbon monoxide and hydrogen.

The process of the present invention is more specifically explained hereinafter.

In the first step for synthesis of a rhodium carbonyl compound, the rhodium carbonyl compound is prepared by reacting the spent hydroformylation reaction medium with carbon monoxide and oxygen under pressure.

In this step, oxygen facilitates the conversion of a deactivated rhodium compound, which is difficult to be converted into a rhodium carbonyl compound. The ratio of partial pressures of carbon monoxide to oxygen employed in this step can be preferably in the range of 5:1 to 30:1 and a total pressure of carbon monoxide and oxygen is preferably 1.01325 to 10.1325 MPa (10 to 100 atmospheres). The partial pressure ratio of carbon monoxide to oxygen which is smaller than 5:1 may cause a danger of explosion, and the partial pressure ratio of carbon monoxide to oxygen which is greater than 30:1 may decrease an effect of the oxygen. The reaction can be conveniently practised at the temperature of 60°C to 150°C, preferably 80°C to 120°C, for 10 minutes to 5 hours.

The first step for synthesis of the rhodium carbonyl compound can be generally practised only using the spent hydroformylation reaction medium itself in the absence of any additional solvent. However, when the spent hydroformylation reaction medium is highly viscous and therefore, is necessarily diluted, a hydroxyl group-containing compound can be used as a solvent.

After reaction of the first step is sufficiently practiced, the reaction mixture containing the produced rhodium carbonyl compound is subjected, without isolation of the rhodium carbonyl compound, to the second step for synthesis of the hydridocarbonyl tris(triorganophosphorus)rhodium in the same reactor.

The second step for synthesis of the hydridocarbonyl tris (triorganophosphorus)rhodium is carried out by adding a hydroxyl group-containing compound and a triorganophosphorus ligand to the reaction mixture containing the rhodium carbonyl compound obtained from the first step and then reacting the mixture with an alkali metal hydroxide and formaldehyde under refluxing condition (about 80^{o}C) to produce the hydridocarbonyl tris(triorganophosphorus)rhodium.

The hydroxyl group-containing compound used in the second step is an alcohol having 1 to 3 carbon atoms, for example, methanol, ethanol, isopropanol, etc., with the ethanol being most preferable. Although the amount of the hydroxyl group-containing compound used in this step is varied depending on the amount and viscosity of the spent hydroformylation reaction medium, the amount of the hydroxyl group-containing compound is generally 0.5 to 20 times, preferably 1 to 10 times, the amount of the spent hydroformylation reaction medium on the basis of weight.

Specific examples of the alkali metal hydroxide which can be used in the second step include sodium hydroxide(NaOH), potassium hydroxide(KOH), lithium hydroxide(LiOH), etc., with the potassium hydroxide being most preferable. In this step, the alkali metal hydroxide is reacted with the hydroxyl group-containing compound to furnish hydrogen to the rhodium compound and can also convert the carbonyl group-containing compound in the spent hydroformylation reaction medium into the hydroxy compound to make the recovery of the desired product easy due to the decrease in the solubility of the resulting hydridocarbonyl tris(triorganophosphorus) rhodium. In addition, since the alkali metal hydroxide can convert the rhodium complex deactivated by a strong binding compound into a easily revivable form, it causes an increase in the recovery of hydridocarbonyl tris(triorganophosphorus)rhodium. The amount of alkali metal hydroxide used in the second step is generally 1 to 40 times, preferably 4 to 20 times, on the basis of rhodium moles .

The formaldehyde which is employed in the present invention is substantially free of water and is generally used in the form of a mixture with methanol. In general, when formaldehyde is used in the form of a mixture with methanol, the weight ratio of methanol : formaldehyde is from 5:1 to 1:1. The amount of formaldehyde used in this reaction can be varied depending on the amount of the spent hydroformylation reaction medium and the rhodium concentration, and is generally selected such that the neat formaldehyde accounts for 0.1 to 10 wt% of the spent hydroformylation reaction medium. The formaldehyde is a source of carbonyl groups in the rhodium compound.

The triorganophosphorus ligand used in the present invention is defined as having a general formula of PX₃ (wherein X is OPh, OR, R, or Ph, in which Ph is phenyl and R is alkyl). The most preferred triorganophosphorus ligand is triphenylphosphine, triphenylphosphite, and the like. The amount of triorganophosphorus ligand used in the present invention can be varied depending on the rhodium content in the spent hydroformylation reaction medium as well as the solubility of ligand in the spent hydroformylation reaction medium and the used hydroxyl group-containing compound. However, the ligand should be used in an excessive molar amount greater than 3 times, preferably from 3 to 30 times, more preferably from 4 to 20 mole times, based on the mole number of rhodium.

The third step of the process according to the present invention is a step for post-treatment of the reaction product obtained from the second step with carbon monoxide and hydrogen to prepare the desired hydridocarbonyl tris(triorganophosphorus) rhodium compound in a high yield.

The post-treatment step should be carried out by treating the reaction product from the second reaction step first with carbon monoxide-containing gas and then with hydrogen. According to this step, the desired hydridocarbonyl tris(triorganophosphorus)rhodium compound which cannot be dissolved in the spent hydroformylation reaction medium and the hydroxyl group-containing compound can be obtained in a high yield.

In the third step, carbon monoxide serves as a source of carbonyl groups to the unreacted rhodium compounds which are caused by insufficient addition of carbonyl groups in the previous steps. The carbon monoxide used in this step can be either carbon monoxide itself or a carbon monoxide-containing gas. As the carbon monoxide-containing gas, synthesis gas comprising carbon monoxide and hydrogen in the ratio of 1:5 to 5:1, preferably 1:1, is generally used. The treatment with the carbon monoxide-containing gas can be conducted at the temperature of 50°C to 100°C, preferably 55°C to 70°C, under the pressure of 0.101325 to 10.1325 MPa (1 to 100 atmospheres), preferably 1.01325 to 4.053 MPa (10 to 40 atmospheres). In this case, the reaction generally requires several minutes to several hours and preferably 0.5 to 3 hours.

Finally, the carbon monoxide-treated product is then necessarily subjected to a hydrogen treatment to obtain a stable hydridocarbonyl tris(triorganophosphorus)rhodium compound.

It is generally known that hydridocarbonyl tris(triorganophosphorus)rhodium can be reversibly converted into a triorganophosphorus ligand and carbon monoxide under carbon monoxide atmosphere as shown in the following reaction scheme [II]: 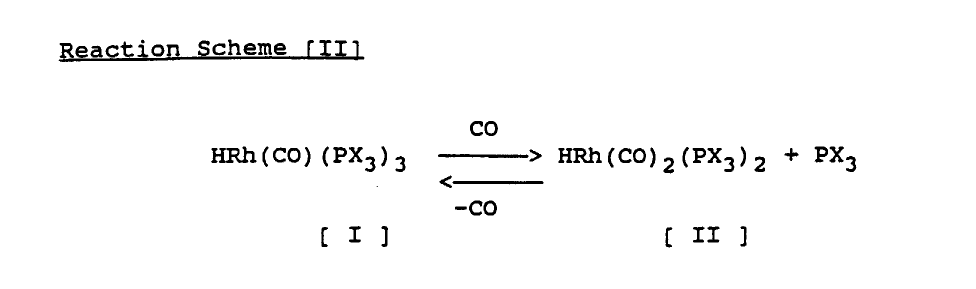 The compound [II] produced in the above reaction [II] can be in a state of equilibrium as shown in the following reaction scheme [III]:

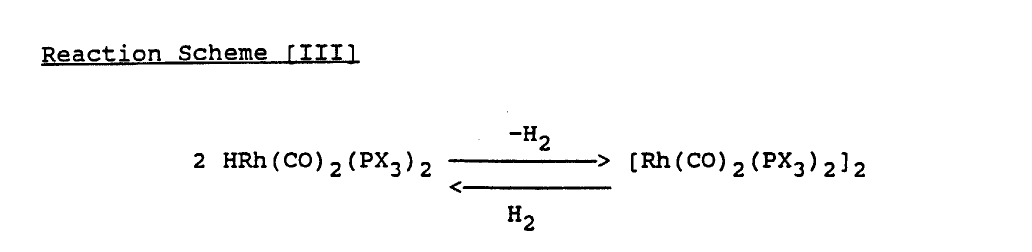

According to the reaction [III] above, a rhodium dimer compound which can be dissolved in the hydroxyl group-containing compound and the spent hydroformylation reaction medium is produced and the formation of such rhodium dimer compound can cause a decrease in recovery of the hydridocarbonyl tris(triorganophosphorus)rhodium compound. Accordingly, in order to obtain the hydridocarbonyl tris(triorganophosphorus)rhodium in a high yield, a hydrogen treatment should be conducted in the presence of an excessive amount of the triorganophosphorus ligand.

The hydrogen treatment can be conveniently carried out at the temperature of from 50°C to 100°C, preferably 60°C to 80°C, under the pressure of from 0.101325 to 10.1325 MPa (1 to 100 atmospheres), preferably 1.01325 to 4.053 MPa (10 to 40 atmospheres). The reaction time of hydrogen treatment is generally several minutes to several hours and preferably 0.5 to 4 hours.

As explained above, the present invention relates to a process for direct preparation of hydridocarbonyl tris(triorganophosphorus)rhodium in a high yield from the spent hydroformylation reaction medium containing the deactivated rhodium catalyst, according to an organic one-phase reaction, which comprises converting the deactivated rhodium catalyst into the rhodium carbonyl compound which is then reacted, without isolation from the reaction solution, directly with the hydroxyl group-containing compound, the alkali metal hydroxide and the triorganophosphorus ligand and subsequently subjected to the post-treatment with carbon monoxide and hydrogen.

The present invention will be more specifically illustrated by the following examples. However, it should be understood that the present invention is not limited to those examples in any manner.

In the examples, the preparedhydridocarbonyl tris(triorganophosphorus)rhodium compounds were identified by means of infrared spectrometry (IR) and X-ray diffraction (XRD) and the recovery rate of hydridocarbonyl tris(triorganophosphorus)rhodium complex was determined by means of inductively coupled plasma (ICP).

### EXAMPLE 1

A spent hydroformylation reaction medium obtained from a continuous hydroformylation process of propylene to produce butyraldehyde in the presence of a rhodium complex catalyst and a triphenylphosphine ligand, which contains the deactivated rhodium catalyst which is determined as having no further commercial value, was distilled to obtain the concentrated distillation residue having a rhodium concentration of about 20,000 ppm. The obtained concentrated distillation residue was aerated at a temperature of 90°C for 24 hours. After the aeration is completed, the aerated distillation residue was analyzed by gas chromatography to identify that all of triphenylphosphine was converted into triphenylphosphine oxide. About 130g of the aerated rhodium-containing distillation residue was added together with 350g of ethanol to a 2L autoclave. The autoclave was charged with about 0.547 MPa (5.4 atmospheres) of oxygen and then charged with carbon monoxide to about 2.685 MPa (26.5 atmospheres) under room temperature. The reaction mixture was heated with stirring and allowed to react at 100°C for 3 hours and then cooled down to room temperature and vented. About 40g of triphenylphosphine was added to the reaction mixture and then about 8g of potassium hydroxide(KOH) and about 10 ml of formaldehyde/methanol solution (1:1 wt%) were added thereto under a refluxing condition (80°C). The reaction mixture was allowed to react for 3 hours and then cooled down to room temperature. Then, the reaction mixture was pressurized to 2.634 MPa (26 atmospheres) with carbon monoxide and allowed to react at 60°C for 2 hours and then carbon monoxide was discharged from the reaction vessel. Subsequently, the reaction vessel was charged with hydrogen to 2.432 MPa (24 atmospheres) and the reaction mixture was allowed to react at 70°C for 2 hours and then cooled down to room temperature. The reaction mixture was filtered to obtain a yellow solid product which was dried in a vaccum dryer. The obtained yellow solid product was identified as hydridocarbonyl tris(triphenylphosphine)rhodium by infrared spectrometry (IR) and X-ray diffraction (XRD). The recovery rate of rhodium as the solid product HRh(CO)(PPh₃)₃ was 95.5% as determined by inductively coupled plasma (ICP) analysis for the solid product and the filtrate.

### EXAMPLE 2

A spent hydroformylation reaction medium as used in Example 1 was distilled to obtain the concentrated distillation residue having the rhodium concentration of 4,200ppm which was then aerated at a temperature of 90°C for 15 hours. About 50g of the aerated distillation residue was added to a 300 ml autoclave. The autoclave was charged with oxygen to about 0.547 MPa (5.4 atmospheres) and then pressurized with carbon monoxide to about 2.685 MPa (26.5 atmospheres). The reaction mixture was heated with stirring and allowed to react at 100°C for 4 hours. The mixture was cooled down to room temperature and then vented. About 10g of triphenylphosphine and 70g of ethanol were added to the reaction mixture and heated up to a reflux condition. Under this condition, 2g of KOH and 10 ml of HCHO/MeOH solution (1:1 wt%) were added to the reaction mixture and then allowed to react for 3 hours.

The reaction vessel was then cooled down to room temperature and pressurized to 2.634 MPa (26 atmospheres) with carbon monoxide. The reaction mixture was allowed to react at 60°C for 2 hours. The reaction vessel was then vented by discharging carbon monoxide and charged with hydrogen to 2.432 MPa (24 atmospheres). The reaction mixture was allowed to react at 70°C for 2 hours, cooled down to room temperature and then filtered to obtain a yellow solid product which was dried in a vaccum dryer. The obtained yellow solid product was identified as hydridocarbonyl tris(triphenylphosphine)rhodium by infrared spectrometry (IR) and X-ray diffraction (XRD). The recovery rate of rhodium as the solid product HRh(CO)(PPh₃)₃ was 95.2% as determined by inductively coupled plasma (ICP) analysis for the solid product and the filtrate.

### EXAMPLE 3

According to the same procedure as Example 2, except that a 300 ml autoclave containing 50g of the aerated distillation residue was charged with oxygen to about 0.274 MPa (2.7 atmosphere) and then pressurized with carbon monoxide to about 2.685 MPa (26.5 atmosphere), a yellow solid product was obtained.

The obtained yellow solid product was identified as hydridocarbonyl tris(triphenylphosphine)rhodium by IR and XRD. The recovery rate of rhodium was 91.8% as determined by ICP analysis for the solid product and the filtrate.

### EXAMPLE 4

A spent hydroformylation reaction medium as used in Example 1 was distilled to obtain the concentrated distillation residue having the rhodium concentration of 4,200ppm which was then aerated at temperature of 90°C for 15 hours. About 300g of the aerated distillation residue was added together with 300g of ethanol to a 2L autoclave. The autoclave was charged with oxygen to about 0.547 MPa (5.4 atmospheres) and then pressurized with carbon monoxide to about 2.685 MPa (26.5 atmospheres), at a room temperature. The reaction mixture was heated with stirring and allowed to react at 100°C for 2.5 hours. The mixture was cooled down to room temperature and then vented. About 40g of triphenylphosphine was added to the reaction mixture and then 10g of potassium hydroxide(KOH) and 10 ml of formaldehyde(HCHO)/methanol(MeOH) solution (1:1 wt%) were added thereto under a refluxing condition (80°C) and then allowed to react for 3 hours.

The reaction vessel was then cooled down to room temperature and pressurized to 2.634 MPa (26 atmospheres) with carbon monoxide. The reaction mixture was allowed to react at 60°C for 2 hours. The reaction vessel was then vented by discharging carbon monoxide and charged with hydrogen to 2.432 MPa (24 atmospheres). The reaction mixture was allowed to react at 70°C for 2 hours, cooled down to room temperature and then filtered to obtain a yellow solid product which was dried in a vacuum dryer. The obtained yellow solid product was identified as hydridocarbonyl tris(triphenylphosphine) rhodium by infrared spectrometry (IR) and X-ray diffraction (XRD). The recovery rate of rhodium was 94.1% as determined by inductively coupled plasma (ICP) analysis for the solid product and the filtrate.

## Claims

1. A process for the direct preparation of hydridocarbonyl tris(triorganophosphorus)rhodium compounds according to an organic one-phase reaction from a distillation residue of a hydroformylation reaction mixture containing a deactivated rhodium complex catalyst, which comprises the steps of :
i) oxidizing said distillation residue with an oxidizing agent;
ii) converting the rhodium comple-x catalyst into a rhodium carbonyl compound in the presence of a gas mixture of carbon monoxide and oxygen;
iii) reacting the obtained rhodium carbonyl compound, without isolation from the reaction mixture, directly with a hydroxyl group-containing compound, an alkali metal hydroxide, formaldehyde and a triorganophosphorus ligand to prepare the hydridocarbonyl tris(triorganophosphorus) rhodium; and
iv) reacting remaining rhodium carbonyl compound with carbon monoxide, and reacting the hydrido carbonyl tris (triorganophosphorus) rhodium with hydrogen.

2. The process according to in claim 1, wherein in the step i) the oxidizing agent is air or oxygen and the oxidation is conducted at a temperature of from 60°C to 120°C under the pressure of from 0.101325 to 1.01325 MPa (1 to 10 atmospheres).

3. The process according to claim 1, wherein the rhodium concentration in the distillation residue is from 1,500 ppm to 30,000 ppm.

4. The process according to claim 1, wherein the step ii) is carried out under a partial pressure ratio of carbon monoxide to oxygen of from 5:1 to 30:1 and a total pressure of carbon monoxide and oxygen of 1.01325 to 10.1325 MPa (10 to 100 atmospheres).

5. The process according to claim 4, wherein in the step ii) the conversion reaction is conducted at a temperature of from 80°C to 120°C.

6. The process according to claim 1, wherein in the step iii) the hydroxyl group-containing compound is ethanol.

7. The process according to claim 6, wherein the hydroxyl group-containing compound is used in an amount of 1 to 10 times the amount of the used distillation residue on the basis of weight.

8. The process according to claim 1, wherein in the step iii) the alkali metal hydroxide is potassium hydroxide.

9. The process according to claim 8, wherein the alkali metal hydroxide is present in an amount of 4 to 20 mole times the that of the total rhodium moles present in said distillation residue.

10. The process according to claim 1, wherein in the step iii) the formaldehyde consists essentially of a water free methanol/formaldehyde mixture which has a ratio from 5:1 to 1:1 and wherein the mixture is used in an amount of from 0.1 to 10 wt%, on the basis of the distillation residue.

11. The process according to claim 1, wherein in the step iii) the triorganophosphorus ligand is triphenylphosphine or triphenylphosphite.

12. The process according to claim 11, wherein the triorganophosphorus ligand is used in a amount of 4 to 20 mole times that of the total rhodium moles present in said distillation residue.

13. The process according to claim 1, wherein step iv) comprises reacting with hydrogen, after reacting with carbon monoxide.

14. The process according to claim 13, wherein the carbon monoxide reaction is conducted at a temperature of from 55°C to 70°C under the pressure of 0.101325 to 10.1325 MPa (1 to 100 atmospheres).

15. The process according to claim 13, wherein the hydrogen reaction is conducted at a temperature of from 60°C to 80°C under the pressure of 0.101325 to 10.1325 MPa (1 to 100 atmospheres).

## Patentansprüche

1. Verfahren zur direkten Herstellung von Hydridocarbonyltris(triorganophosphor)-Rhodium-Verbindungen mittels einer organischen Einphasenreaktion aus einem Destillationsrückstand eines Hydroformylierungsreäktionsgemisches, das einen deaktivierten Rhodiumkomplex-Katalysator enthält, das die Schritte:
i) Oxidieren des besagten Destillationsrückstandes mit einem Oxidationsmittel;
ii) Umwandeln des Rhodiumkomplex-Katalysators in eine Rhodiumcarbonylverbindung in Anwesenheit eines Gasgemisches aus Kohlenmonoxid und Sauerstoff;
iii) Reaktion der erhaltenen Rhodiumcarbonylverbindung ohne Isolierung aus dem Reaktionsgemisch direkt mit einer hydroxylgruppenhaltigen Verbindung, einem Alkalimetallhydroxid, Formaldehyd und einem Triorganophosphor-Liganden, um das Hydridocarbonyltris(triorganophosphor)-Rhodium herzustellen und
iv) Reaktion der verbliebenen Rhodiumcarbonylverbindung mit Kohlenmonoxid und Reaktion des Hydridocarbonyltris(triorganophosphor)-Rhodiums mit Wasserstoff umfaßt.

2. Verfahren nach Anspruch 1, dadurch gekennzeichnet, daß im Schritt i) das Oxidationsmittel Luft oder Sauerstoff ist und die Oxidation bei einer Temperatur von 60 °C bis 120 °C unter dem Druck von 0,101325 bis 1,01325 MPa (1 bis 10 atm) durchgeführt wird.

3. Verfahren nach Anspruch 1, dadurch gekennzeichnet, daß die Rhodiumkonzentration im Destillationsrückstand von 1500 ppm bis 30000 ppm ist.

4. Verfahren nach Anspruch 1, dadurch gekennzeichnet, daß der Schritt ii) unter einem Partialdruckverhältnis von Kohlenmonoxid zu Sauerstoff von 5:1 bis 30:1 und einem Gesamtdruck von Kohlenmonoxid und Sauerstoff von 1,01325 bis 10,1325 MPa (10 bis 100 atm) durchgeführt wird.

5. Verfahren nach Anspruch 4, dadurch gekennzeichnet, daß im Schritt ii) die Umwandlungsreaktion bei einer Temperatur von 80 °C bis 120 °C durchgeführt wird.

6. Verfahren nach Anspruch 1, dadurch gekennzeichnet, daß im Schritt iii) die hydroxylgruppen-haltige Verbindung Ethanol ist.

7. Verfahren nach Anspruch 6, dadurch gekennzeichnet, daß die hydroxylgruppen-haltige Verbindung in einer Menge des 1- bis 10fachen der Menge des verwendeten Destillationsrückstandes auf Gewichtsbasis verwendet wird.

8. Verfahren nach Anspruch 1, dadurch gekennzeichnet, daß im Schritt iii) das Alkalimetallhydroxid Kaliumhydroxid ist.

9. Verfahren nach Anspruch 8, dadurch gekennzeichnet, daß das Alkalimetallhydroxid in einer Molmenge des 4- bis 20fachen der Gesamtrhodiummole vorliegt, die in besagtem Destillationsrückstand vorliegen.

10. Verfahren nach Anspruch 1, dadurch gekennzeichnet, daß im Schritt iii) das Formaldehyd aus im wesentlichen einem wasserfreien Methanol/Formaldehyd-Gemisch besteht, das ein Verhältnis von 5:1 bis 1:1 hat, und daß das Gemisch in einer Menge von 0,1 bis 10 Gewichtsprozent auf der Basis des Destillationsrückstandes verwendet wird.

11. Verfahren nach Anspruch 1, dadurch gekennzeichnet, daß im Schritt iii) der Triorganophosphorligand Triphenylphosphin oder Triphenylphosphit ist.

12. Verfahren nach Anspruch 11, dadurch gekennzeichnet, daß der Triorganophosphorligand in einer Molmenge des 4- bis 20fachen der Gesamtrhodiummole, die in besagtem Destillationsrückstand vorliegen, verwendet wird.

13. Verfahren nach Anspruch 1, dadurch gekennzeichnet, daß Schritt iv) die Reaktion mit Wasserstoff nach der Reaktion mit Kohlenmonoxid umfaßt.

14. Verfahren nach Anspruch 13, dadurch gekennzeichnet, daß die Kohlenmonoxidreaktion bei einer Temperatur von 55 °C bis 70 °C unter dem Druck von 0,101325 bis 10,1325 MPa (1 bis 100 atm) durchgeführt wird.

15. Verfahren nach Anspruch 13, dadurch gekennzeichnet, daß die Wasserstoffreaktion bei einer Temperatur von 60 °C bis 80 °C unter dem Druck von 0,101325 bis 10,1325 MPa (1 bis 100 atm) durchgeführt wird.

## Revendications

1. Procédé pour la préparation directe d'hydrurocarbonyl-tris(triorganophosphore)rhodiums selon une réaction dans une seule phase organique à partir d'un résidu de distillation d'un mélange réactionnel d'hydroformylation contenant un complexe de rhodium catalytique désactivé, qui comprend les étapes suivantes :
i) oxyder ledit résidu de distillation avec un agent oxydant ;
ii) convertir le complexe de rhodium catalytique en un rhodium-carbonyle en présence d'un mélange gazeux constitué de monoxyde de carbone et d'oxygène ;
iii) faire réagir directement le rhodium-carbonyle obtenu, sans l'isoler du mélange réactionnel, avec un composé contenant un groupe hydroxyle, un hydroxyde de métal alcalin, du formaldéhyde et un ligand triorganophosphoré pour préparer l'hydruro-carbonyl-tris-(triorganophosphore) rhodium ; et
iv) faire réagir le rhodium-carbonyle restant avec du monoxyde de carbone, et faire réagir l'hydrurocarbonyl-tris(triorganophosphore) rhodium avec de l'hydrogène.

2. Procédé selon la revendication 1, dans lequel, dans l'étape i), l'agent oxydant est l'air ou l'oxygène et l'oxydation est conduite à une température de 60°C à 120°C sous une pression de 0,101325 à 1,01325 MPa (1 à 10 atmosphères).

3. Procédé selon la revendication 1, dans lequel la concentration de rhodium dans le résidu de distillation est de 1500 ppm à 30 000 ppm.

4. Procédé selon la revendication 1, dans lequel l'étape ii) est conduite à un rapport de pressions partielles du monoxyde de carbone à l'oxygène de 5:1 à 30:1 et une pression totale de monoxyde de carbone et d'oxygène de 1,01325 à 10,1325 MPa (10 à 100 atmosphères).

5. Procédé selon la revendication 4, dans lequel, dans l'étape ii), la réaction de conversion est conduite à une température de 80°C à 120°C.

6. Procédé selon la revendication 1, dans lequel, dans l'étape iii), le composé contenant un groupe hydroxyle est l'éthanol.

7. Procédé selon la revendication 6, dans lequel le composé contenant un groupe hydroxyle est utilisé en une quantité de 1 à 10 fois la quantité du résidu de distillation utilisé, sur base pondérale.

8. Procédé selon la revendication 1, dans lequel, dans l'étape iii), l'hydroxyde de métal alcalin est l'hydroxyde de potassium.

9. Procédé selon la revendication 8, dans lequel l'hydroxyde de métal alcalin est présent en une quantité molaire de 4 à 20 fois le nombre total de moles de rhodium existant dans ledit résidu de distillation.

10. Procédé selon la revendication 1, dans lequel, dans l'étape iii), le formaldéhyde consiste essentiellement en un mélange anhydre de méthanol/formaldéhyde en un rapport de 5:1 à 1:1, et dans lequel le mélange est utilisé en une quantité de 0,1 à 10 % en poids par rapport au résidu de distillation.

11. Procédé selon la revendication 1, dans lequel, dans l'étape iii), le ligand triorganophosphoré est la triphénylphosphine ou le phosphite de triphényle.

12. Procédé selon la revendication 11, dans lequel le ligand triorganophosphoré est utilisé en une quantité molaire de 4 à 20 fois le nombre total de moles de rhodium existant dans ledit résidu de distillation.

13. Procédé selon la revendication 1, dans lequel l'étape iv) comprend la réaction avec l'hydrogène après la réaction avec le monoxyde de carbone.

14. Procédé selon la revendication 13, dans lequel la réaction avec le monoxyde de carbone est conduite à une température de 55°C à 70°C sous une pression de 0,101325 à 10,1325 MPa (1 à 100 atmosphères).

15. Procédé selon la revendication 13, dans lequel la réaction avec l'hydrogène est conduite à une température de 60°C à 80°C sous une pression de 0,101325 à 10,1325 MPa (1 à 100 atmosphères).
